Europäisches Patentamt

European Patent Office

Office européen des brevets

(19)

(11) Numéro de publication: **0 198 770**
**A2**

## (12) DEMANDE DE BREVET EUROPEEN

(21) Numéro de dépôt: **86400777.8**

(22) Date de dépôt: **11.04.86**

(51) Int. Cl.⁴: **G01N 33/49**

(30) Priorité: **11.04.85 FR 8505472**

(43) Date de publication de la demande:
**22.10.86 Bulletin 86/43**

(84) Etats contractants désignés:
**AT BE CH DE GB IT LI LU NL SE**

(71) Demandeur: **Vieillard, Didier**
**Polyclinique du Parc Route d'Assevent**
**F-59600 Maubeuge(FR)**

(72) Inventeur: **Vieillard, Didier**
**Polyclinique du Parc Route d'Assevent**
**F-59600 Maubeuge(FR)**

(74) Mandataire: **Picard, Jean-Claude Georges et al**
**Cabinet Plasseraud 84, rue d'Amsterdam**
**F-75009 Paris(FR)**

(54) **Procédé et appareil pour la mesure de la coagulation du plasma sanguin.**

(57) L'invention concerne un procédé et un appareil de mesure de la coagulation du sang. Le principe en est de détecter, dès le début de la coagulation, l'arrêt d'un (1), la détection s'effectuant à l'aide de cellules photo-électriques ou analogues (15,16). Un calculateur (17) permet d'effectuer automatiquement la mesure des taux de coagulation et de les afficher. Les corpuscules peuvent être des bulles de gaz - (12) microscopiques, ou les grains d'une poudre à haut degré de mouillabilité.

EP 0 198 770 A2

## Procédé et appareil pour la mesure de la coagulation du plasma sanguin

La présente invention concerne un procédé et un appareil pour la mesure de la coagulation du plasma sanguin.

Comme on le sait, la coagulation est le changement d'état physique du plasma, qui est initialement liquide et qui se transforme en gel lors de la polymérisation du fibrinogène.

Les procédées ou appareils actuellement connus, utilisés pour la détection de ce changement d'état, sont essentiellement de deux types : photométriques, ou mécaniques.

Dans les procédés photométriques, on mesure, par exemple à l'aide d'une source lumineuse et d'une cellule, l'opacité du plasma, car celle-ci augmente au moment de la coagualtion. Toutefois, il n'y a pas coïncidence exacte entre les deux phénomènes, et la mesure ne peut donc pas être très précise.

Dans les procédé dits mécaniques, on détecte les variations des propriétés mécaniques du plasma, qui accompagnent la coagulation.

Dans certains appareils, un barreau magnétique soumis au champ d'un aimant extétieur mobile est placé au fond du tube de mesure contenant le plasma. On détecte l'arrêt de la rotation ou des oscillations du barreau, qui se produit au moment de la coagulation.

·Dans d'autres appareils, au contraire, un aimant extérieur maintient une bille immobile au fond d'un tube tournant, et l'on détecte l'entraînement de la bille, qui se produit au moment de la coagulation.

Ces procédés mécaniques, dans lesquels on met à profit l'augmentation de la résistance mécanique d'un caillot en cours de coagulation, sont eux aussi insuffisamment précis, car il faut que la coagulation soit relativement avancée pour que l'on constate une augmentation notable de cette résistance mécanique.

Il y a lieu de noter en outre que ces procédés ne peuvent être valablement exploités dans le cas d'un plasma dilué, car les phénomènes d'ordre mécanique indiqués plus haut sont encore moins nets.

Le but de la présente invention est de remédier à ces divers inconvénients des procédés connus, et en particulier d'augmenter la précision de la mesure. Il convient aussi de mettre en oeuvre un principe de mesure facilement automatisable.

A cet effet, un procédé pour la mesure de la coagulation du plasma sanguin est, conformément à la présente invention, essentiellement caractérisé en ce que l'on établit dans le tube un courant de corpuscules, et en ce que l'on mesure l'espace de temps entre une référence de temps déterminée et l'instant auquel le courant de corpuscules est stoppé du fait de la coagulation, ledit espace de temps étant représentatif du taux de coagulation.

Bien entendu, on prendra comme référence de temps un instant significatif, qui sera en règle générale l'instant auquel le réactif approprié est introduit dans le tube.

Ce nouveau principe de mesure s'est montré apte à fournir des mesures emtrêment précises et fidèles, car l'arrêt du courant des corpuscules peut être constaté exactement au moment du début du processus de coagulation. Ce courant peut être un courant descendant dans le tube sous l'effet de la pesanteur. Dans ce cas, on utilise avantageusement comme corpuscules les grains d'une poudre à haut degré de mouillabilité, introduite à la partie supérieure du· tube, les dimensions de ces grains, par exemple en verre, pouvant être extrêmement réduites, de l'ordre de quelques microns, de sorte qu'ils soient immédiament arrêtés par le réseau de fibrine dès le début de la coagulation, même dans un sang dilué.

Selon une variante du procédé, mettant en oeuvre globalement le même principe, on peut prévoir encore que lesdits corpuscules sont des bulles de gaz et que l'on établit à cet effet dans le fond du tube un débit de gaz comprimé, de sorte à créer une série de bulles de très faible diamètre en ascension dans ledit plasma.

Là encore, ce principe de mesure s'est montré apte à fournir des mesures extrêmement précises et fidèles, car l'arrêt de l'ascension des bulles peut encore être constaté exactement au moment du début du processus de coagulation. Il est en effet possible de créer au fond du plasma des bulles de gaz suffisamment fines, par exemple d'un diamètre de l'ordre de 30 à 60 $\mu$, pour qu'elles soient arrêtées instantanément par le réseau de fibrine, dès sa formation, laquelle marque précisément le début de la coagulation.

L'arrêt des corpuscules (grains de poudre ou bulles) pourra mesurer l'espace de temps en question avec un chronomètre, et noter à chaque gois le résultat.

Toutefois, il sera avantageux de procéder de façon automatique, surtout dans le cas d'un grande nombre de tests, grâce à un appareil pour la mise en oeuvre du procédé défini plus haut.

L'invention concerne donc encore un appareil de mesure de la coagulation du plasma sanguin, essentiellement caractérisé en ce qu'il comporte : au moins un tube de mesure associé à moyens adaptés à y introduire automatiquement le plasma et un réactif en un instant déterminé servant de référence de temps ; des moyens d'introduction desdits corpuscules dans le tube de sorte à y créer un courant desdits corpuscules ; des moyens de détection de l'arrêt dudit courant ; et des moyens de mesure du temps écoulé entre ladite référence de temps et ledit arrêt.

Dans le cas où les corpuscules sont des bulles de gaz, un tel appareil comportera des moyens d'introduction d'un gaz comprimé, par un passage ultrafin, dans le fond dudit tube et des moyens de détection de l'arrêt de l'ascension de la série de bulles ainsi créées dans le tube.

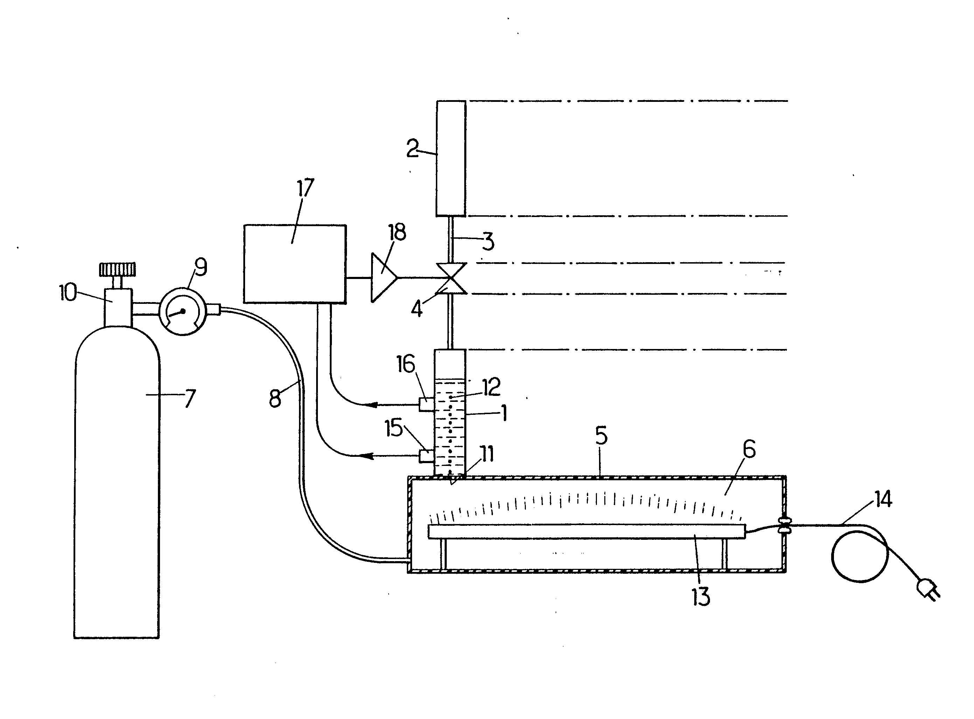

Un mode de réalisation d'un tel appareil va maintenant être décrit à titre d'exemple nullement limitatif, avec référence à la figure unique du dessin ci-annexé, qui en est une représentation - schématique.

Sur cette figure, on n'a représenté, pour simplifier, qu'un seul tube de mesure 1 d'une série de tubes pouvant en comporter un nombre quelconque. De même, on n'a représenté que les moyens associés à ce tube, pour l'introduction automatique du plasma et des réactifs, ce moyens pouvant être les mêmes pour tous les tubes 1 de la série, et être d'ailleurs commandés individuellement, par groupes, ou encore tous simultanément. Ces moyens peuvent comprendre par exemple un tube d'alimentation 2 en communcation avec le tube de mesure 1 correspondant par une conduite 3 portant une vanne propre à être commandée automatiquement, par exemple une électrovanne 4. Le tube 2 peut recevoir successivement le plasma et le réactif, mais on peut aussi utiliser deux tubes 2 séparés, pour déverser successivement le plasma et le réactif dans le tube de mesure 1.

La série de tubes 1, qui sont thermostatés à 37°C, repose sur la paroi supérieure 5 d'une enceinte 6 reliée à une bouteille d'air comprimé 7 par une conduite 8. Un manomètre 9 placé à la sortie du détendeur 10 de la bouteille permet de contrôler la pression de l'air injecté dans l'enceinte 6. Cette pression pourra être de l'ordre de 200 cm de colonne d'eau.

L'enceinte 6 communique avec le fond de chaque tube 1 par un trou ultrafin 11 traversant la paroi 5, de sorte qu'une série de bulles microscopiques 12 s'élève dans le mélange plasma-réactif introduit dans le tube 1.

Le choix du diamètre des trous 11 est très important, car si ce diamètre est trop grand les bulles seront trop grosses et ne seront pas immédiatement stoppées dès l'apparition du réseau de fibrine. Par contre, si ce diamètre est trop faible, l'arrêt des bulles sera plus difficile à détecter, les trous seront extrêmement difficiles à élaborer, et leur risque de bouchage sera trop grand.

Un diamètre de trou 11 conciliant ces divers impératifs sera d'environ 50 $\mu$.

Pour que les bulles 12 soient faciles à détecter, soit à l'oeil nu, soit à l'aide de tout appareillage approprié, l'enceinte 6 contient une rampe d'éclairage 13 pouvant être branchée sur le secteur par un cordon 14 et éclairant les bulles 12 par les trous 11.

Pour la détection des bulles, on pourra utiliser, dans l'appareil automatique présentement décrit, et pour chaque tube 1, une ou plusieurs cellules photoélectriques. Sur la figure, on a représenté deux cellules 15 et 16 espacées sur le tube 1, ce qui, par différence des temps $\delta t$ de passage permet de contrôler la vitesse des bulles 12. Dès que cette vitesse tombe à zéro (arrêt des bulles), au temps $t_1$, on peut obtenir le temps $\Delta$ de coagulation, par différence avec le temps $t_0$ auquel le réactif a été introduit dans le tube 1, par commande de l'ouverture de l'électrovanne 4 : $\Delta t = t_1 - t_0$.

A partir de cette mesure du temps de coagulation, un bloc électronique à microprocesseur 17, auquel sont reliées les cellules photo-électriques 15 et 16, peut calculer les facteurs de coagulation, pour en exprimer le taux en % de la normale, ceci tube par tube ou simultanément pour toute la série de tubes.

La précision de la mesure du $\Delta t$ est excellente, de l'ordre du 1/10e de s pour un temps de coagulation de 10 à 40 secondes.

ON a également représenté sur la figure un amplificateur 18, alimenté par un signal du microprocesseur, et grâce auquel l'ouverture de l'électrovanne 4 peut, comme indiqué plus haut, être commandée automatiquement à l'instant de référence $t_0$.

Le microprocesseur peut bien entendu aussi assurer toutes les autres commandes séquentielles nécessaires, la mise en mémoire des mesures, un affichage numérique des résultats, la régulation des différents paramètres physiques des différents tests, etc.

Les principaux tests qu'un appareil conforme à la présente invention permet d'effectuer sont les suivants :

-taux de prothrombine,

-temps de céphaline kaolin,

-dosage des facteurs I, II, V, VII, VIII, IX,

-temps de Howell,

-dosage de l'anthithrombine III.

Pour le temps de Howell, on a constaté qu'un appareil conforme à l'invention permettait de le mesurer avec une aussi bonne précision qu'en manuel, ce qui n'est pas le cas des appareils connus jusqu'à présent.

Comme il va de soi et comme il résulte d'ailleurs déjà de ce qui précède, l'invention ne se limite nullement à ceux de ses modes d'application et de réalisation qui ont été plus particulièrement envisagés ; elle en embrasse, au contraire, toutes les variantes.

Par exemple, il est bien entendu que les électrovannes telles que 4 pourraient être remplacées par tout autre dispositif de type médical approprié, par exemple par des vannes de type péristaltique.

Dans le cas où l'on met en oeuvre un courant de corpuscules qui ne sont pas des bulles de gaz, mais des grains de poudre, l'appareil pourra être globalement semblable à celui qui vient d'être décrit, mais avec des aménagements, notamment pour l'alimentation des tubes en poudre, par exemple en billes de verre miscroscopiques, et éventuellement quant aux moyens de détection. En tout cas, il n'y aura plus lieu de prévoir une enceinte alimentée en air comprimé, et la nécessité d'élaborer des trous miscroscopiques dans une plaque disparaît également, ce qui peut être avantageux du point de vue économique.

On utilisera de préférence une poudre de densité voisine de celle de l'eau, à haut degré de mouillabilité et réfrigente, comme le verre, de sorte que le tout début de la coagulation --même d'un sang très dilué--pourra être détecté. L'utilsation de la poudre permet donc d'envisager d'effectuer, et de façon autmatique, de nouveaux types d'analyses, nécessitant des moyens de mesure très fins.

## Revendications

1. Procédé pour la mesure de la coagulation du plasma sanguin, le plasma étant disposé, avec un ou plusieurs réactifs, dans au moins un tube de mesure, caractérisé en ce que l'on établit dans le tube un courant de corpuscules, et en ce que l'on mesure l'espace de temps entre une référence de temps déterminée et l'instant auquel le courant de corpuscules est stoppé du fait de la coagulation, ledit espace de temps étant représentatif du taux de coagulation.

2. Procédé selon la revendication 1, caractérisé en ce que lesdits corpuscules sont les grains d'une poudre à haut degré de mouillabilité, introduite à la partie supérieure du tube.

3. Procédé selon la revendication 1, caractérisé en ce que lesdits corpuscules sont des bulles de gaz, et en ce que l'on établit à cet effet dans le fond du tube un débit de gaz comprimé, de sorte à créer une série de bulles de très faible diamètre en ascension dans ledit plasma.

4. Procédé selon la revendication 3, caractérisé en ce que l'on crée dans le plasma des bulles de gaz d'un diamètre de l'ordre de 30 à 60 $\mu$ environ.

5. Appareil pour la mise en oeuvre d'un procédé conforme à l'une quelconque des revendications précédentes, caractérisé en ce qu'il comporte : au moins un tube de mesure associé à des moyens adaptés à y introduire automatiquement le plasma et un réactif en un instant déterminé servant de référence de temps ; des moyens d'introduction desdits corpuscules dans le tube de sorte à y créer un courant desdits corpuscules ; des moyens de détection de l'arrêt dudit courant ; et des moyens de mesure du temps écoulé entre ladite référence de temps et ledit arrêt.

9. Appareil selon la revendication 5, caractérisé en ce qu'il comporte des moyens (7,8) d'introduction d'un gaz comprimé, par un passage ultrafin (11), dans le fond dudit tube (1) et des moyens de détection de l'arrêt de l'ascension de la série de bulles ainsi créées dans le tube (1).

7. Appareil selon la revendication 6, caractérisé en ce que lesdits moyens d'introduction d'un gaz comprimé dans le fond de chaque tube de mesure (1) comprennent une enceinte (6) de communication avec une source de gaz comprimé (7) et dont une paroi (5) est percée de trous ultrafins - (11), chaque trou communiquant avec le fond d'un tube de mesure (1).

8. Appareil selon la revendication 7, caractérisé en ce que lesdits trous (11) ont un diamètre de l'ordre de 30 à 60 $\mu$ environ.

9. Appareil selon l'une quelconque des revendications 5 à 8, caractérisé en ce que lesdits moyens de détection comprennent au moins une cellule photoélectrique (15) associée à chaque tube de mesure (1).

10. Appareil selon la revendication 9, caractérisé en ce que lesdits moyens de détection comprennent au moins deux cellules photo-électriques (15,16) associées à chaque tube de mesure (1) et espacées le long de celui-ci.

11. Appareil selon l'une quelconque des revendications 7 à 10, caractérisé en ce que ladite enceinte - (6) contient une rampe d'éclairage (13).

12. Appareil selon l'une quelconque des revendications 5 à 11, caractérisé en ce qu'il comporte un bloc électronique de mesure, de calcul, de commande et d'enregistrement (17), propre à effectuer les mesures de temps, le calcul des facteurs et taux de coagulation ou autres paramètres, ainsi que leur mise en mémoire et éventuellement leur affichage, de même qu'à assurer les différentes commandes séquentielles nécessaires, notamment la commande automatique des moyens précités (2-4) d'introduction du plasma et des réactifs dans les tubes de mesure (1).